Europäisches Patentamt

European Patent Office

Office européen des brevets

(19)

(11) Numéro de publication: **0 253 037 B1**

(12) **FASCICULE DE BREVET EUROPEEN**

(45) Date de publication de fascicule du brevet:
03.07.91

(51) Int. Cl.5: **A61K 33/16, A61K 7/18,**
**A61K 7/48, A61K 7/00,**
**A01N 59/10**

(21) Numéro de dépôt: 86402716.4

(22) Date de dépôt: 08.12.86

(54) Composition inhibitrice d'au moins un être vivant unicellulaire et/ou virus, procédé de fabrication et applications de cette composition.

(30) Priorité: 22.05.86 FR 8607310

(43) Date de publication de la demande:
20.01.88 Bulletin 88/03

(45) Mention de la délivrance du brevet:
03.07.91 Bulletin 91/27

(84) Etats contractants désignés:
AT BE CH DE ES FR GB GR IT LI LU NL SE

(56) Documents cités:
EP-A- 0 055 109     EP-A- 0 162 574
EP-A- 0 226 187     FR-A- 1 297 708
FR-A- 1 468 676     FR-A- 2 431 859
US-A- 3 647 700     US-A- 3 995 029
US-A- 3 996 350     US-A- 4 097 590
US-A- 4 359 475     US-A- 4 368 186
US-A- 4 473 547

DICTIONNAIRE VIDAL, 1985, page 582, O.V.P.,
Paris, FR; "Fluor monal"

DICTIONNAIRE VIDAL, 1986, pages
640,1086,1467, O.V.P., Paris, FR

(73) Titulaire: **ATLANTIC PHARMACEUTICAL PRO-**
**DUCTS LIMITED**
**Celbridge**
**Dunaghcumper Co Kildare(IE)**

(72) Inventeur: **Bourbon, Pierre**
**36, rue Volta**
**F-31000 Toulouse(FR)**
Inventeur: **Billot, Pierre**
**20, Boulevard de la Saussaye**
**F-92200 Neuilly Sur Seine(FR)**
Inventeur: **Lagny, Pierre**
**192, The Grove**
**Celbridge Co Kildare(IE)**

(74) Mandataire: **Derambure, Christian**
**BUGNION ASSOCIES 55, rue Boissonade**
**F-75014 Paris(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention concerne l'inhibition ou destruction des êtres vivants unicellulaires tels que protozoaires, microbes, bactéries, gamètes, champignons ou autres et des virus. Elle vise donc en particulier les domaines techniques de la contraception locale, de l'antibiothérapie, de l'antisepsie, que ce soit dans le cadre de la pharmacie ou de la cosmétique, ainsi que de la désinfection.

Dans toute la suite, on entend par "inhibition" d'un être vivant unicellulaire ou d'un virus le fait, soit d'empêcher sa prolifération, soit de le rendre incapable d'effectuer certaines fonctions qu'il effectue habituellement. On entend par "destruction" le fait de tuer les être vivants unicellulaires ou virus.

Dans le cadre de l'invention, et dans toute la suite, on entend par "substance" tout composé chimique ou association de composés chimiques ayant au moins une fonction donnée ou commune aux composés et susceptible de rentrer dans la composition d'un produit fini, généralement en association avec un excipient et éventuellement d'autres substances. De même, le terme "produit" désigne un produit fini utilisable. Généralement, un produit fini est donc constitué d'au moins un excipient et de plusieurs substances, chaque substance étant constituée d'un ou plusieurs composés chimiques ayant des fonctions similaires ou identiques. Ce terme "substance" peut correspondre à une réalité pratique, mais peut aussi être purement théorique et fonctionnel dans les cas de mélanges complexes où les composés ont des actions multiples ou interférant les unes avec les autres. Cette classification fonctionnelle en composés, substances, produits, ne correspond pas nécessairement au procédé de fabrication du produit et aux mélanges effectivement réalisés en pratique. Le terme "composition" est employé ici et dans toute la suite pour désigner une substance pharmaceutique ou cosmétique, de façon usuelle.

On connaît déjà des substances inhibitrices ou destructrices d'êtres vivants unicellulaires, que ce soit dans les domaines biologique, pharmaceutique, cosmétique, ou même dans des domaines plus généraux tels que la désinfection ou autres.

On connaît en particulier des brevets américains 4 339 441 et 4 359 475 des compositions spermicides.

On connaît aussi de nombreux composés chimiques pouvant être utilisés dans les méthodes de contraception locale pour l'être vivant. En particulier, on sait que les tensio-actifs surfactifs (par exemple les ammoniums quaternaires, les nonoxynols, les dérivés d'amido-éthylglycinate sur acides gras, le méthyltauride d'oxyde sel sodique ...) et certains autres composés tels que le nitrate de phénylmercure, le para-menthanylphénylpolyoxyéthylène éther ou le sel trisodique de l'éther sulfurique de polysaccharide, ont des actions inhibitrices ou destructrices d'êtres vivants unicellulaires, notamment spermicides.

On connaît également des substances agissant mécaniquement sur les spermatozoïdes, par exemple en les immobilisant. Le brevet américain 4 368 186 décrit de tels effets, et plus particulièrement l'association de "poloxamer" à des inhibiteurs de spermatozoïdes, afin de maîtriser la gelification et la solubilisation des produits et d'augmenter ainsi mécaniquement en addition ou en synergie l'efficacité des inhibiteurs.

On connaît également des compositions contraceptives locales, notamment spermicides, renfermant de tels composés chimiques, et pouvant être utilisées localement pour inhiber le pouvoir reproducteur de gamètes, notamment de spermatozoïdes de l'être humain ou animal. Ces compositions connues comportent une certaine concentration de composé chimique inhibiteur supérieure à la concentration minimale inhibitrice, ou C.M.I., en solution ou suspension dans un excipient pharmaceutique qui dépend de la forme galénique utilisée. On sait en effet que pour obtenir la mort de la totalité des spermatozoïdes contenus dans 0,2 millilitres de sperme en moins de 5 secondes (conditions du test spermicide total selon les normes de l'IPPF, Fédération Internationale pour le Planning Familial), il est nécessaire que la concentration du principe actif spermicide dans 1 millilitre de composition pharmaceutique soit supérieure ou égale à la concentration minimale inhibitrice, ou CMI, de ce principe actif. La CMI d'un composé chimique dépend de ce composé chimique, mais aussi des conditions dans lesquelles il est utilisé, c'est-à-dire des formes galéniques qui renferment la composition.

On connaît plus généralement des compositions pharmaceutiques ou cosmétiques renfermant au moins un principe actif de base inhibiteur ou destructeur d'au moins un être vivant unicellulaire et des médicaments ou cosmétiques renfermant de telles compositions.

Dans le domaine de l'antisepsie et de la désinfection, on connaît déjà de nombreux principes actifs : halogènes (dérivés chlorés ou iodés...), aldéhydes, alcools, phénols, acides, métaux (sels d'argent, de cuivre, de zinc, de mercure...), amidines, biguanides, carbanilides, oxydants (eau oxygénée, permanganates de potassium...), colorants, agents tensio-actifs et mouillants (cationiques, anioniques, amphotères ou organiques).

On connaît également des produits antiseptiques et/ou désinfectants et/ou antiprotozoaires et/ou antifongiques et/ou antibiotiques et/ou antiviraux renfermant de telles substances inhibitrices ou destructrices, ainsi que des procédés de fabrication de ces substances ou compositions.

Les brevets américains US-A-4 097 590, US-A-3 996 350, et US-A-3 995 029 décrivent l'utilisation de fluorure de sodium avec, en proportion mineure par rapport au fluorure, un agent mouillant constitué d'un tensio-actif anionique pour soigner l'acné et la mycose du pied d'athlète.

Les demandes de brevet européen EP-A-0 226 187 et PCT WO-A-87 03482, opposables au titre de l'article 54(3) uniquement, décrivent un produit pharmaceutique destiné à une application topique pour combattre le virus de l'herpès qui contient un acide benzoïque, du phénol et un fluorure alcalin.

Les substances ou compositions connues donnent en général satisfaction mais posent des problèmes pratiques d'utilisation.

Les problèmes posés par ces substances ou compositions connues sont principalement les suivants : Tout d'abord on désire généralement avoir une efficacité totale d'inhibition ou de destruction du (ou des) être(s) vivant(s) unicellulaire(s) visé(s) ou des virus, c'est-à-dire une inhibition ou une destruction de 100 % dans les conditions pratiques d'utilisation. Or, ce n'est pas toujours le cas si l'on ne prend pas des précautions d'utilisation astreignantes, ou si l'on est obligé de limiter les doses de principe actif (cas de la cosmétique par exemple).

De plus, on désire généralement avoir une action sur un ou plusieurs êtres vivants unicellulaires ou virus donnés, par exemple de gamètes ou des êtres pathogènes, sans provoquer de réactions secondaires indésirables à court terme et/ou à long terme (action sur d'autres êtres vivants unicellulaires, irritations, endommagement de l'environnement ...). Or, on constate en pratique que les doses nécessaires pour résoudre le premier problème sus-cité provoquent des réactions secondaires à long terme (généralement liées à une utilisation régulière), voire même à court terme (utilisation au coup par coup). Dans le domaine médical, on a constaté, à partir de certains doses seuils et pour certains principes actifs, des effets tératogènes et/ou cancérigènes. Dans le domaine de la désinfection, on peut aussi constater des effets indésirables vis-à-vis des matériaux à désinfecter.

L'invention a pour objet d'augmenter le pouvoir inhibiteur ou destructeur d'une substance ou composition renfermant une concentration donnée limitée de principe actif de base inhibiteur ou destructeur, sans augmenter cette concentration. Un autre objet de l'invention est de réduire les concentrations en principes actifs de base sans diminuer pour autant le pouvoir inhibiteur ou destructeur de la substance ou composition, et ce afin d'éviter les réactions secondaires et de mieux contrôler la sélectivité par rapport aux différents êtres vivants unicellulaires et virus.

En particulier, l'invention vise à proposer une composition pharmaceutique ou cosmétique, notamment contraceptive locale, et un médicament ou produit renfermant une telle composition, qui puisse être utilisé soit au coup par coup, soit de façon régulière et continue à long terme, sans le risque d'effet secondaire , et particulier tératogène ou cancérigène, et dont l'efficacité est totale c'est-à-dire dont le pourcentage d'échecs statistiquement est nul ou négligeable, et ce dans des conditions habituelles simples d'utilisation (sans précautions particulières).

De plus, l'invention se propose de fournir un produit pharmaceutique contraceptif local, facile à utiliser et totalement efficace, c'est-à-dire donnant des résultats comparables à ceux de la contraception orale, sans en avoir les inconvénients.

L'invention a aussi pour but de proposer un composé chimique utilisable dans une méthode contraceptive locale.

Enfin, l'invention a aussi pour objet de réduire les doses de principes actifs inhibiteurs ou destructeurs d'êtres vivants unicellulaires ou virus dans les médicaments, les cosmétiques ou les désinfectants, notamment les antiseptiques, antibiotiques, bactéricides, antiprotozoaires, antifongiques, spermicides, antiviraux...

Pour ce faire, l'invention concerne l'application d'au moins un principe actif inhibiteur ou destructeur d'au moins un être vivant unicellulaire ou virus et d'un principe activateur dans des proportions appropriées pour inhiber ou détruire au moins un enzyme associé audit être vivant unicellulaire ou virus pour obtenir un produit ou un médicament destiné à inhiber ou détruire cet être vivant unicellulaire ou virus, à l'exception du cas de l'association de l'acide benzoïque, du phénol et d'un fluorure alcalin en solution aqueuse ou hydroalcoolique pour obtenir un produit destiné à inhiber ou détruire le virus de l'herpès (demandes de brevet EP-A-0 226 187 et WO-A-87 03482 opposables au titre de l'article 54 (3)).

Pour ce faire, l'invention propose une substance inhibitrice ou destructrice d'au moins un être vivant unicellulaire -notamment protozoaire, microbe, bactérie, gamète, champignon ou autre et virus-, caractérisée en ce qu'elle comporte au moins un principe actif de base inhibiteur ou destructeur dudit être vivant unicellulaire et un principe inhibiteur ou destructeur d'au moins un enzyme associé audit être vivant, constituant un activateur -notamment agissant par synergie- du principe actif de base.

De préférence, le principe inhibiteur ou destructeur d'au moins un enzyme associé, appelé dans toute la suite principe " activateur" est un agent empêchant le fonctionnement du couple enzyme/substrat.

Or, il a été constaté avec surprise qu'un tel agent pouvait être avantageusement constitué de l'anion fluorure $F^-$ émis à partir d'un composé fluoré, soit spontanément, soit après action enzymatique . On a aussi constaté qu'un tel composé fluoré a lui-même un pouvoir inhibiteur ou destructeur des êtres vivants unicellulaires et des virus et que ce composé fluoré agit en synergie avec le principe actif de base associé.

L'invention propose en particulier une composition pharmaceutique et une composition cosmétique comportant en combinaison au moins un principe actif de base et un composé chimique fluoré émetteur de l'anion fluorure $F^-$ dont la fonction est d'augmenter l'efficacité de l'action du (ou des) principe(s) actif(s) de base.

Plus précisément, l'invention propose une composition contraceptive locale, notamment spermicide, caractérisée en ce qu'elle renferme, à titre de principe actif inhibiteur ou destructeur de gamètes, direct ou par potentialisation, au moins un composé chimique émetteur de l'anion fluorure $F^-$, et un excipient.

L'invention propose également un médicament, un produit antiprotozoaire -notamment antiprotozoaire local- et/ou bactéricide -notamment bactéricide local-, et/ou antifongique -notamment antifongique local- et/ou antibiotique -notamment antibiotique local et/ou antiviral-, un produit cosmétique -notamment cosmétique local tel que savon synthétique ou lait-, un produit antiseptique et/ou désinfectant -notamment antiseptique local- et un produit contraceptif appliqué localement au contact de gamètes -notamment ovule, crème, gelée, solution, mousse, comprimé, film soluble, tampon, suppositoire vaginal-, caractérisés en ce qu'ils comportent une substance ou une composition selon l'invention.

Ici, et dans toute la suite, le terme "local" signifie utilisé à un endroit déterminé à traiter, par exemple le vagin ou autres parties anatomiques, de sorte que l'action se produit dans l'environnement proche de cet endroit à l'encontre des êtres vivants unicellulaires qui viennent en contact avec le produit et ce contrairement aux utilisations par voie générale (par exemple par voie orale).

Une substance, une composition, un médicament, un produit, un composé chimique selon l'invention concernent aussi bien les applications par voie locale que les applications par voie générale. Par exemple, un spermicide selon l'invention est appliqué localement. Par ailleurs, pour surmonter les effets de résistance des êtres vivants unicellulaires pathogènes aux thérapeutiques habituelles, on utilisera préférentiellement l'invention par voie générale.

L'invention propose aussi l'application dans un produit cosmétique d'une composition renfermant au moins un principe actif de base et un composé chimique fluoré émetteur de l'anion fluorure $F^-$ en tant que principe activateur du (ou des) principe(s) actif(s) de base; l'application d'un composé chimique fluoré émetteur de l'anion fluorure $F^-$ pour fabriquer une substance renfermant au moins un principe actif de base, en tant que principe activateur du (ou des) principe(s) actif(s) de base; l'application d'une substance ou composition selon l'invention pour obtenir un médicament destiné à une utilisation à titre d'antibiotique et/ou antiprotozoaire et/ou bactéricide et/ou antifongique et/ou antiviral; et l'application d'une substance ou composition selon l'invention pour obtenir un contraceptif local pour l'être humain ou animal, notamment inhibiteur ou destructeur de gamètes.

L'invention propose également un procédé de fabrication d'une substance ou composition selon l'invention, inhibitrice ou destructrice d'au moins un être vivant unicellulaire -notamment microbe, bactérie, gamète, champignon, ou autre virus - caractérisé en ce qu'on mélange au moins un principe actif de base inhibiteur ou destructeur dudit être vivant unicellulaire avec un principe inhibiteur ou destructeur d'un système enzymatique associé audit être vivant, -notamment un composé fluoré émetteur de l'anion fluorure $F^-$-, constituant un activateur du principe actif de base.

L'invention propose un composé chimique renfermant du fluor ionisable pour son utilisation dans une méthode contraceptive locale pour l'être vivant, notamment dans un produit contraceptif local pour l'être humain ou animal selon l'invention ou dans une composition selon l'invention.

Selon l'invention, le principe activateur inhibiteur ou destructeur d'au moins un enzyme associé à un être vivant unicellulaire permet, en "bloquant" le couple enzyme/substrat, de sensibiliser notablement ledit être vivant unicellulaire aux agressions extérieures, notamment à l'action dudit principe actif de base inhibiteur ou destructeur dudit être vivant unicellulaire. On sait en effet que chaque être vivant unicellulaire possède un système enzymatique vital. Par ailleurs, les êtres vivants unicellulaires peuvent émettre, selon les circonstances (par exemple résistance à antibiotique), des enzymes particuliers permettant la destruction d'un agent agresseur (lactamase détruisant les pénicillines), dits "enzymes protecteurs". L'invention consiste donc à inhiber le rôle de ces enzymes et donc de fragiliser l'être vivant unicellulaire en créant les conditions optimales d'efficacité des principes actifs à son encontre. Cela présente de nombreux avantages par rapport à l'art antérieur, à savoir, d'une part, de satisfaire les buts préalablement annoncés et, d'autre part, de pouvoir agir de façon plus sélective, sur un être vivant unicellulaire déterminé ou une famille déterminée d'êtres vivants unicellulaires en agissant sur un enzyme déterminé ou une famille déterminée d'enzymes. Dans le cas des virus, l'invention a pour but d'inhiber les enzymes nécessaires a leur formation et/ou à leur réplication. Dans toute la suite, "enzymes associés" désigne les enzymes vitaux et/ou protecteurs de l'être vivant unicellulaire et/ou des virus.

Les inventeurs ont déterminé que le fluor à l'état ionisé F⁻ agit de façon particulièrement efficace et bénéfique en tant que principe activateur agissant à l'encontre des enzymes associés et ce sous des concentrations minimes courantes. On remarquera avec avantage que le fluor ionisé F⁻ est très courant, économique et d'emploi relativement simple. Ses manipulations et utilisations sont bien maîtrisées, ainsi que les réactions secondaires qu'il est susceptible d'engendrer en fonction des doses employées.

L'invention propose enfin un procédé d'inhibition ou de destruction d'au moins un être vivant unicellulaire -notamment protozoaire, microbe, bactérie, gamète, champignon ou autre, virus-, caractérisé en ce qu'on utilise au moins un principe actif de base inhibiteur ou destructeur dudit être vivant unicellulaire, et un principe inhibiteur ou destructeur d'au moins un enzyme associé audit être vivant, constituant un activateur -notamment agissant par synergie- du principe actif de base, et un tel procédé dans lequel on utilise au moins une substance ou composition selon l'invention.

D'autres caractéristiques et avantages de l'invention apparaîtront à la lecture de la description détaillée et des exemples qui suivront.

L'invention concerne l'inhibition ou la destruction d'êtres vivants unicellulaires tels que les protozoaires, microbes, bactéries, gamètes, champignons, qu'ils soient pathogènes ou non et de virus. L'invention trouve essentiellement deux types distincts d'applications : soit des applications dans le domaine cosmétique ou pharmaceutique (cas des exemples suivants de spermicides ou de bactéricides agissant contre des agents pathogènes), soit des applications dans des domaines plus courants tels que l'agriculture, la désinfection (exemple suivant) ou autres. Dans tous les cas, une substance selon l'invention est caractérisée en ce qu'elle comporte au moins un principe actif de base inhibiteur ou destructeur dudit être vivant unicellulaire et un principe inhibiteur ou destructeur d'au moins un enzyme associé audit être vivant ou virus constituant un activateur -notamment par synergie lorsqu'il est lui-même également inhibiteur ou destructeur dudit être vivant-du principe actif de base.

La fonction du principe activateur est donc d'annihiler le rôle du système enzymatique associé au(x)dit(s) être(s) vivant(s) ou virus. Pour ce faire, les inventeurs ont déterminé que ce principe activateur est avantageusement un agent empêchant le fonctionnement du couple enzyme/substrat, notamment l'anion fluorure F⁻ émis à partir d'un composé fluoré par exemple un dérivé métallique du fluor. Des résultats très positifs ont été observés avec le fluorure de sodium, le fluorure de calcium, le fluorure de potassium, le fluorure d'aluminium, le fluorure d'étain, le fluorure d'ammonium, le monofluorophosphate de sodium.

Les êtres vivants unicellulaires ou virus suivants peuvent, par exemple, être concernés par l'invention :

. gamètes (spermatozoïdes, ovules)
. coques à Gram positif, notamment staphylocoques et streptocoques
. coques à Gram négatif, notamment gonocoques
. bacilles à Gram positif
. bacilles à Gram négatif, notamment colibacilles ou Escherichia coli
. bacilles acido-alcoolo-résistants, notamment mycobactéries telles que mycobactérium smegmatis.
. bactéries de forme spiralée, notamment spirochètes, tels que treponema
. bactéries diverses, notamment chlamydia
. protozoaires flagellés, notamment trichomonas
. levures diverses, notamment candida albicans
. virus ou rétrovirus, notamment LAV ou HIV ("human immunodeficiency virus"), et Herpès.

Les enzymes suivants associés à au moins un de ces êtres vivants unicellulaires ou virus peuvent être, par exemple, concernés par l'invention (Tableau 1) :

T A B L E A U   1

| ENZYME | SUBSTRAT |
| --- | --- |
| Phosphatase alcaline | 2- naphtyl phosphate |
| Estérase (C 4) | 2- naphtyl butyrate |
| Estérase Lipase (C 8) | 2- naphtyl caprylate |
| Lipase (C 14) | 2- naphtyl myristate |
| Leucine arylamidase | L- leucyl -2- naphtylamide |
| Valine arylamidase | L- valyl -2- naphtylamide |
| Cystine arylamidase | L- cystyl -2- naphtylamide |
| Trypsine | N- benzoyl -DL- arginine -2- naphtylamide |
| α Chymotrypsine | N- glutaryl-phénylalanine -2- naphtylamide |
| Phosphatase acide | 2- naphtyl phosphate |
| Naphtol -AS-Bl- phosphohydrolase | Naphtol -AS-Bl- phosphate |
| α galactosidase | 6-Br-2- naphtyl α D- galactopyranoside |
| β galactosidase | 2- naphtyl -βD- galactopyranoside |
| β glucuronidase | naphtol-AS-Bl-βD- glucuronide |
| α glucosidase | 2- naphtyl -αD- glucopyranoside |
| β glucosidase | 6-Br-2- naphtyl-βD- glucopyranoside |
| N- acétyl -β glucosaminidase | 1- naphtyl-N-acétyl-βD- glucosamimnide |
| α mannosidase | 6-Br-2- naphtyl-αD- mannopyranoside |
| α fucosidase | 2- naphtyl-αL-fucopyranoside |

Un principe actif de base utilisé dans une substance ou composition selon l'invention est par exemple un tensio-actif surfactif anionique, cationique, amphotère ou non-ionique, notamment un ammonium quaternaire. Un exemple de tensio-actif surfactif cationique est le chlorure de benzalkonium ou autre alkylbenzalkonium. Un exemple de tensio-actif surfactif anionique est un méthyltauride d'oxyde sel sodique. Un exemple de tensio-actif surfactif amphotère est un dérivé d'amidoéthylglycinate sur acide gras. Un exemple de tensio-actif surfactif non ionique est un nonoxynol. Le principe actif de base peut aussi avantageusement être du nitrate de phénylmercure ou du para-menthanylphénylpolyoxyéthylène, éther, ou un sel trisodique de l'éther sulfusique de polysaccharide, ou autre (halogène, aldéhyde, alcool, phénol, acide, métal, amidine, biguamide, carbanilide, oxydant, colorant ...)

Dans les différentes applications testées, on a pu mettre en évidence le fait que le principe activateur seul a généralement aussi une activité directement à l'encontre dudit être vivant unicellulaire ou virus. On a pu alors constater avec surprise que l'association du principe activateur au principe actif de base a un effet synergique, dans la mesure où les résultats obtenus ne correspondent pas seulement à la somme des résultats escomptés par la seule présence du principe actif de base et du principe activateur, mais sont au contraire supérieurs à cette somme.

L'efficacité de tels principes actifs ou activateurs peut se mesurer grâce à la concentration minimale inhibitrice ou CMI qui correspond à la concentration de principe actif provoquant, en un temps donné, l'inhibition ou la mort de la totalité des êtres vivants unicellulaires ou virus.

Avantageusement, dans une substance selon l'invention, la concentration en anion fluorure $F^-$ émise par le composé fluore activateur est inférieure à la concentration minimale inhibitrice (CMI) de l'anion fluorure $F^-$ sans principe actif de base.

De même, la concentration du principe actif de base dans une substance selon l'invention peut être inférieure à la concentration minimale inhibitrice (CMI) de ce principe actif de base en l'absence de principe activateur. Une telle substance selon l'invention a quand même, et de façon surprenante une efficacité totale, c'est-à-dire au moins égale à celle des substances comportant soit uniquement l'anion fluorure $F^-$ à une concentration supérieure ou égale à sa CMI, soit uniquement un principe actif de base à une concentration supérieure ou égale à sa CMI.

Par ailleurs, le principe actif de base peut lui-même aussi être émetteur de principe activateur, notamment d'anion fluorure $F^-$. Une substance ou composition selon l'invention peut renfermer plusieurs principes actifs de base dont les fonctions peuvent être identiques ou différentes et/ou plusieurs principes activateurs agissant sur au moins un enzyme associé à un ou à plusieurs de ces principes actifs de base.

Une substance ou composition selon l'invention peut faire l'objet de multiples applications pour obtenir des produits, administrables, selon les utilisations desdits produits, localement ou par voie générale.

Parmi les utilisations particulièrement avantageuses de produits selon l'invention, on peut citer les différentes utilisations thérapeutiques possibles, à titre de médicaments. En effet, on rencontre le problème de l'inhibition ou de la destruction des êtres vivants unicellulaires ou virus dans un nombre considérable et toujours croissant d'applications thérapeutiques. Il peut s'agir de la lutte contre les agents pathogènes, et les produits sont alors des antibiotiques et/ou antiprotozoaires et/ou bactéricides et/ou antifongiques et/ou antiseptiques et/ou antiviraux ou même contre des agents non pathogènes, tels que les gamètes, par exemple les spermatozoïdes dans le cadre de la contraception, notamment la contraception locale.

Une substance ou composition selon l'invention peut aussi avantageusement être appliquée dans un produit cosmétique, pour des doses et concentrations qui ne permettent pas de le classer dans le domaine pharmaceutique.

En effet, par exemple, les concentrations efficaces de composés chimiques fluorés et même de principes actifs de base peuvent être dimunuées dans des proportions telles qu'elles deviennent inférieures aux valeurs seuils de concentrations séparant les domaines pharmaceutique et cosmétique, sans pour autant diminuer l'efficacité du produit.

Une substance ou composition selon l'invention peut aussi être appliquée dans des produits ni cosmétiques, ni pharmaceutiques, par exemple dans le domaine de l'agriculture comme antifongique, antiprotozoaire, antiseptique, antibiotique, ou autre, ou encore dans le domaine de la désinfection de surfaces.

Le mode de réalisation préférentiel de l'invention actuellement connu est celui des produits utilisés localement sur les parties génitales des mammifères mâles et/ou femelles, à titre de spermicides et/ou de bactéricides pour lutter contre les maladies sexuellement transmissibles (MST).

Les principes actifs préférentiellement utilisés sont le chlorure de benzalkonium et le nonoxynol 9. Le principe activateur préférentiellement utilisé est un dérivé métallique quelconque du fluor, par exemple le fluorure de sodium. Toutes les formes galéniques connues sont utilisables, à savoir notamment ovule, crème, gelée, solution, mousse, comprimé, film soluble, tampon, suppositoire vaginal ou autre.

Les proportions à utiliser de principe actif et de principe activateur varient d'une forme galénique à une autre, puisque seules les concentrations effectives résultant in vivo sont importantes vis-à-vis de l'efficacité du produit.

Ces proportions doivent varier entre les CMI et les concentrations maximales à partir desquelles on induit des effets secondaires. En application locale, on doit éviter les intolérances (irritations) des parties traitées. En application générale, on doit éviter les concentrations toxiques.

Ainsi, la concentration in vivo de chlorure de benzalkonium est préférentiellement de 1,2 % (en poids) et la concentration in vivo d'anion fluorure F⁻ est préférentiellement de 0,5 % (en poids).

On va maintenant décrire plusieurs modes de réalisation préférentiels de l'invention, pour diverses applications testées, en se référant à des essais effectués in vitro et in vivo pour différents êtres vivants unicellulaires.

I) APPLICATION DE L'INVENTION POUR LA CONTRACEPTION LOCALE :

L'invention concerne avantageusement un produit pharmaceutique spermicide appliqué localement de façon qu'il soit en contact avec le sperme et qu'il tue ou inhibe les spermatozoïdes.

Un produit pharmaceutique spermicide selon l'invention peut se présenter sous différentes formes galéniques : comprimé, ovule, solution, crème, gelée, film soluble, tampon, mousse, suppositoire vaginal, introduit dans le vagin avant l'acte sexuel de façon à tuer ou inhiber les spermatozoïdes, avant qu'ils n'entrent en contact avec les ovules, pour éviter la fécondation.

Un produit selon l'invention comporte une composition qui, elle, renferme à titre de principe actif spermicide direct, ou par potentialisation, au moins un composé chimique selon l'invention, qui renferme du fluor ionisable, c'est-à-dire émetteur de l'anion fluorure F⁻.

Un composé chimique selon l'invention est émetteur de l'anion fluorure F⁻ lorsqu'il est solvaté, notamment en solution aqueuse. Il est, par exemple, constitué d'un dérivé métallique du fluor, tel que le fluorure de sodium, le fluorure de calcium, le fluorure de potassium, le fluorure d'aluminium, le fluorure d'étain, le fluorure d'ammonium, le mono-fluorophosphate de sodium ..., ou d'un composé organique fluoré tel qu'une amine fluorée.

Pour mesurer l'activité spermicide d'un composé chimique, on peut, par exemple, utiliser le test spermicide total selon les normes de l'IPPF qui consiste à déterminer la concentration minimale inhibitrice ou CMI (exprimée par un pourcentage en masse) de composé chimique dans 1 millilitre de solution provoquant en 5 secondes la mort de la totalité des spermatozoïdes contenus dans 0,2 millilitres de sperme. L'essai est effectué sur au moins 6 spermes de donneurs différents, répondant aux conditions minima suivantes de l'IPPF :
- âge de l'échantillon : deux heures;
- densité au mm³ : 50 millions de spermatozoïdes;
- mobilité : 50 % des spermatozoïdes doivent se mouvoir en avant avec rapidité quant on les examine à 35° - 37° C dans un échantillon frais;
- viscosité : éjaculat convenablement liquéfié, ne filant pas et d'aspect homogène à l'oeil nu;
- recueillis dans des tubes de verre stériles, fermés hermétiquement, conservés à 37° C.

Les inventeurs ont déterminé, dans ces conditions, que l'anion fluorure F⁻, présent au titre de 5 milligrammes par litre, a une activité spermicide de 100 % selon le test de l'IPPF.

Une composition pharmaceutique spermicide selon une première variante possible de l'invention renferme comme unique principe actif inhibiteur ou destructeur au moins un composé chimique fluoré émetteur de l'anion fluorure F⁻. Le titre de F⁻ dans la composition est avantageusement supérieur à 4,5 ppm, notamment de l'ordre de 5 ppm, si l'on veut une composition 100 % inhibitrice.

De plus, les inventeurs ont déterminé que l'anion fluorure F⁻, outre son activité spermicide directe, a une activité spermicide par potentialisation des composés spermicides connus. Par conséquent, une composition selon l'invention est avantageusement constituée en premier lieu d'au moins un principe actif spermicide de base, tel qu'un tensio-actif surfactif anionique, cationique, amphotère ou non-ionique, ou encore du para-menthanylphénylpolyoxyéthylène éther ou un sel trisodique de l'éther sulfurique de polysaccharide ou autre, en second lieu d'au moins un composé fluoré selon l'invention émetteur de l'anion fluorure F⁻, et enfin d'un excipient pharmaceutique et des divers additifs habituels (anti-oxydant, ...)

Dans une composition selon l'invention, la concentration en principe actif de base peut être inférieure à la CMI de ce principe atif en l'absence de l'ion F⁻, la composition étant tout de même inhibitrice à 100 %, c'est-à-dire satisfaisant au test spermicide total de l'IPPF. Une composition selon l'invention peut renfermer un mélange de plusieurs principes actifs de base inhibiteurs de gamètes et/ou un mélange de plusieurs composés chimiques fluorés différents.

Dans toute la suite, les pourcentages donnés sont des pourcenages en poids.

Les modes de réalisation préférentiels de l'invention pour ses applications en contraception locale sont les suivants :

. OVULES :

| | |
|---|---|
| Chlorure de benzalkonium | 1,20 % |
| Anion F⁻ | 0,50 % |
| (par exemple Fluorure de Sodium) | |

Excipients : Glycérides semi synthétiques ou beurre de cacao, ou gélatine, glycérine et eau purifiée, antioxygènes, antiseptiques.

. CREMES et LAITS :

| | |
|---|---|
| Chlorure de benzalkonium | 1,20 % |
| anion F⁻ | 0,50 % |
| (ex : Fluorure de Sodium) | |

Excipients : Eau distillée ou purifiée, humectants, émulsifiant, stabilisateur, antioxygène, antiseptique. (A répartir en proportions variables en fonction de la viscosité et du pH à obtenir).

. ONGUENTS et POMMADES :

| | |
|---|---|
| Chlorure de benzalkonium | 1,20 % |
| Anion F⁻ | 0,50 % |
| (ex : Fluorure de Sodium) | |

Excipients : Eau distillée ou purifiée, émulsifiant, excipients du type corps gras (vaseline, lanoléine, lanovaseline, stéarovaseline) stabilisateur, antioxygène, antiseptique. (A répartir en proportions variables en fonction de la viscosité et du pH à obtenir).

. GELEE :

| | |
|---|---|
| Chlorure de benzalkonium | 1,20 % |
| Anion F⁻ | 0,50 % |
| (par exemple Fluorure de Sodium) | |

Excipients : Dérivés solubles de la cellulose compatibles avec les tensio-actifs surfactifs cationiques, eau distillée ou purifiée, glycérine, sorbitol, antioxygène, antiseptique. (A répartir en proportions variables en fonction de la viscosité et du pH à obtenir).

9

. FILM SOLUBLE :

| | |
|---|---|
| Chlorure de benzalkonium | 1,20 % |
| Anion F⁻ | 0,50 % |
| (par exemple Fluorure de Sodium) | |

Excipients : Alcool polyvinylique, glycérine, sorbitol, propylèneglycol, eau distillée ou purifiée antioxygène.

. COMPRIMES :

| | |
|---|---|
| Chlorure de benzalkonium | 25 mg par comprimé |
| Anion F⁻ | 10 mg par comprimé |
| (par exemple Fluorure de Sodium) | |

Excipients : Lactose, stéarate de magnésium, cellulose, amidon, acide citrique, bicarbonate de sodium.

. SAVONS SYNTHETIQUES :

| | |
|---|---|
| Chlorure de benzalkonium | 2 % |
| Anion F⁻ | 1 % |
| (par exemple Fluorure de sodium) | |

Excipients : Produits moussants et mouillants compatibles avec les ammoniums quaternaires (par exemple tensio-actif amphotère du type bétaine ou amino-bétaine) émoliants, stabilisateur, antioxygène, antiseptique.

. SOLUTIONS :

| | |
|---|---|
| Chlorure de benzalkonium | 0,50 % |
| Anion F⁻ | 0,25 % |
| (par exemple Fluorure de Sodium) | |

Excipients : Eau distillée ou purifiée, éthanol, antioxygène, glycérine, sorbitol, antiseptique. (A répartir en proportions variables en fonction du pH à obtenir).

ESSAI COMPARATIF N° 1 :

On a déterminé, par le test de l'IPPF, les CMI de divers composés spermicides connus. Les résultats suivants ont été obtenus en faisant réagir 1 millilitre de composition sur 0,2 millilitre de sperme et en déterminant la concentration minimale qui entraîne la mort de la totalité des spermatozoïdes en 5 secondes, et ce sur 6 spermes de donneurs différents.

| Composé spermicide | CMI (% en poids) |
|---|---|
| p-menthanylphénylpolyoxyéthylène éther | 0,006 |
| sel trisodique de l'éther sulfurique de polysaccharide | 0,007 |
| tensio-actif surfactif anionique (méthyltauride d'oxyde sel sodique) | 1 |
| tensio-actif surfactif cationique (chlorure de benzalkonium) | 0,006 |
| tensio-actif surfactif non ionique (nonoxynol 9) | 0,006 |
| tensio-actif surfactif amphotère (dérivés d'amidoéthylglycinate sur acides gras) | 0,001 |

ESSAI N° 2 :

Le même test spermicide total de l'IPPF a été effectué avec 1 millilitre de composition contenant déjà 0,0001 % (en poids) d'anion fluorure $F^-$ réagissant sur 0,2 millilitre de sperme, en déterminant la concentration minimale des composés spermicides qui entraîne la mort de la totalité des spermatozoïdes en 5 secondes, et ce sur 6 spermes de donneurs différents.

| Composé spermicide | CMI (% en poids) |
|---|---|
| p-menthanylphénylpolyoxyéthylène éther | 0,003 |
| sel trisodique de l'éther sulfurique de polysaccharide | 0,0025 |
| tensio-actif surfactif anionique (méthyltauride d'oxyde sel sodique) | 0,50 |
| tensio-actif surfactif cationique (chlorure de benzalkonium) | 0,002 |
| tensio-actif surfactif non ionique (nonoxynol 9) | 0,003 |
| tensio-actif surfactif amphotère (dérivés d'amidoéthylglycinate sur acides gras) | 0,0005 |

On remarque ainsi que le pouvoir inhibiteur des principes spermicides connus est de beaucoup amélioré par adjonction de fluor ionisable dans la composition. Une composition selon l'invention pourra donc contenir très peu de principe actif de base spermicide, notamment dans une concentration inférieure à la concentration minimale inhibitrice de ce principe actif en l'absence de l'ion $F^-$.

De plus, la concentration de l'ion $F^-$ associé à un principe actif de base pourra aussi être très faible, notamment inférieure à la concentration minimale inhibitrice de l'anion fluorure $F^-$.

A titre d'exemple, les inventeurs ont déterminé que, si une solution de chlorure de benzalkonium à 0,003 % et une solution renfermant 0,0001 % d'anion fluorure $F^-$ n'ont chacune pas d'activité spermicide totale selon le test de l'IPPF, une solution contenant à la fois 0,003 % de chlorure de benzalkonium et 0,0001 % d'anion fluorure $F^-$ satisfait au test de l'IPPF. On constate donc un effet de synergie.

ESSAI N° 3 :

Cet essai consiste à réaliser un essai identique aux essais N° 1 et 2 ci-dessus mentionnés, mais en présence de borate de sodium, afin de complexer l'ion $F^-$ : l'effet spermicide de l'ion $F^-$ a totalement disparu, démontrant ainsi que c'est uniquement l'ion $F^-$ qui est actif ou potentialisateur de l'effet spermicide.

ESSAI N° 4 :

Dans les mêmes conditions que précédemment décrites (essais N° 1 à 3), différents chlorures de benzalkonium perfluorés ont été utilisés comme composés spermicides de base. On a ainsi montré que les chlorures de benzalkonium perfluorés ont une activité spermicide similaire à celle du chlorure de benzalkonium. D'ailleurs, cette activité demeure identique à elle-même dans les conditions de l'essai N° 3, en présence de borate de sodium, ce qui démontre que le fluor fixé sur le noyau benzénique n'a pas été ionisé.

On a également réalisé l'essai N° 2 sur ces chlorures de benzalkonium perfluorés mélangés avec un autre composé chimique émetteur de l'ion $F^-$. On a constaté également la potentialisation de l'activité spermicide des chlorures de benzalkonium perfluorés. Ce même essai réalisé en présence de borate de sodium (selon l'essai N° 3) a abouti à une activité spermicide qui est celle des chlorures de benzalkonium perfluorés.

Ces deux essais N° 3 et 4 montrent que la potentialisation du principe actif de base n'a lieu qu'en présence de l'anion fluorure $F^-$.

Pour fabriquer un produit pharmaceutique spermicide selon l'invention, on réalise une solution de concentration donnée en anion fluorure $F^-$ et, éventuellement, en principe actif de base, que l'on intègre dans l'excipient choisi selon la forme galénique que l'on veut fabriquer. Dans le cas du comprimé, le principe actif de base et le composé chimique émetteur de l'ion $F^-$ sont intégrés à l'excipient sous forme de produits bruts.

ESSAI COMPARATIF N° 5 :

Cet essai in vitro a été réalisé sur des préparations galéniques renfermant du chlorure de benzalkonium comme principe actif de base, en déterminant après simulation in vitro des conditions réelles in vivo (extraction, solubilisation ...), les CMI de ce chlorure de benzalkonium.

Cet essai réalisé sans principe activateur a conduit aux résultats suivants :

| FORME GALENIQUE | CMI (% en poids) de chlorure de benzalkonium |
|---|---|
| Ovule | 0,0063 |
| Crème | 0,0083 |
| Tampon | 0,0075 |
| Comprimé | 0,0095 |
| Film soluble | 0,0080 |
| Gelée | 0,0080 |

Les pourcentages correspondent aux proportions (en poids) de chlorure de benzalkonium dans la solution in vitro utilisée pour le test spermicide selon l'IPPF, obtenue après simulation et mesurées par titrage à partir d'un échantillon prélevé sur cette solution.

ESSAI N° 6 :

Les mêmes conditions que lors de l'essai comparatif N° 5 ont été utilisées, mais à partir de formes galéniques contenant au départ chacune 0,45 % (en poids) d'anion fluorure F⁻.

Les résultats suivants ont été obtenus :

| FORME GALENIQUE | CMI (% en poids) de chlorure de benzalkonium |
|---|---|
| Ovule | 0,0023 |
| Crème | 0,0030 |
| Tampon | 0,0025 |
| Comprimé | 0,0025 |
| Film soluble | 0,0017 |
| Gelée | 0,0033 |

II) APPLICATION DE L'INVENTION DANS LE DOMAINE DE L'ANTISEPSIE, DE L'ANTIBIOTHERAPIE ... ET NOTAMMENT DANS LA LUTTE CONTRE LES MST :

Les modes de réalisation préférentiels de l'invention par exemple pour son application en dermatologie à titre d'antiseptique sont les suivants :

13

CREMES et LAITS :

Chlorure de benzalkonium              1,20 %

Anion F⁻                                     0,50 %

(par exemple Fluorure de Sodium)

Excipients : Eau distillée ou purifiée, humectants, émulsifiant, stabilisateur, antioxygène, antiseptique. (A répartir en proportions variables en fonction de la viscosité et du pH à obtenir).

ONGUENTS et POMMADES :

Chlorure de benzalkonium              1,20 %

Anion F⁻                                     0,50 %

(par exemple Fluorure de Sodium)

Excipients : Eau distillée ou purifiée, émulsifiant, excipients du type corps gras (vaseline, lanoléine, lanovaseline, stéarovaseline), stabilisateur, antioxygène, antiseptique. (A répartir en proportions variables en fonction de la viscosité et du pH à obtenir).

. SAVONS SYNTHETIQUES :

Chlorure de benzalkonium              2 %

Anion F⁻                                     1 %

(par exemple Fluorure de Sodium)

Excipients : Produits moussants et mouillants compatibles avec les ammoniums quaternaires (par exemple tensio-actif amphotère du type bétaine ou amino-bétaine) émoliants, stabilisateur, antioxygène, antiseptique.

. SOLUTIONS :

Chlorure de benzalkonium              0,50 %

Anion F⁻                                     0,25 %

(par exemple Fluorure de Sodium)

Excipients : Eau distillée ou purifiée, éthanol, antioxygène, glycérine, serbitol, antiseptique. (A répartir en proportions variables en fonction du pH à obtenir).

Les modes de réalisation préférentiels de l'invention, par exemple pour son application en dermatologie à titre d'antibiotique local sont les suivants :

SOLUTIONS:

| | |
|---|---|
| Erythromycine base | 4 % |
| Anion F⁻ | 0,5 % |

Excipients :
Alcool éthylique, propylène glycol, eau distillée.

GELS:

| | |
|---|---|
| Erythromycine base | 4 % |
| Anion F⁻ | 0,5 % |

Excipients : Alcool éthylique, hydroxypropylcellulose, eau distillée, glycérine.

POMMADES :

| | |
|---|---|
| Néomycine base | 0,35 % |
| Anion F⁻ | 0,50 % |
| ou Bacitracine | 50 000 UI % |
| Anion F⁻ | 0,50 % |
| ou Oxytétracycline [chlorhydrate] | 3 % |
| Anion F⁻ | [0,50 %] |
| ou Auréomycine [Chlorhydrate] | [3 %] |
| Anion F⁻ | [0,50 %] |

Excipients : Vaseline, huile de vaseline, lanoline

CREMES:

| | |
|---|---|
| Soframycine sulfate | 2,5 % |
| Anion F⁻ | 0,50 % |

Excipients : Propylèneglycol, polyoxyethylèneglycol, eau distillée.
Les modes de réalisation préférentiels de l'invention, par exemple pour son application en oto-rhino-laryngologie, à titre d'antibiotique local sont les suivants :

.   <u>POMMADES OPHTALMIQUES</u> :

|  |  |
|---|---|
| Auréomycine | 1 % |
| Anion F⁻ | 0,50 % |
| ou Oxytétracycline | 0,50 % |
| Anion F⁻ | 0,25 % |

Excipients : Vaseline, huile de vaseline, lanoline

.   <u>SOLUTIONS NASALES</u>:

|  |  |
|---|---|
| Soframycine sulfate | 1,25 % |
| Anion F⁻ | 0,50 % |

Excipients : Eau distillée, acide citrique, chlorure de sodium.

Pour augmenter l'activité des préparations d'antibiothérapie locale, on peut favorablement ajouter 0,10 % de chlorure de benzalkonium.

Les modes de réalisation préférentiels de l'invention par exemple pour son application en gynécologie et plus particulièrement au titre de la lutte contre les MST sont celles décrites précédemment sous la rubrique "Application pour la contraception locale" et sous les dénominations "ovules", "crèmes", "onguents et pommades", "gelées", "comprimés", "savons synthétiques" et "solutions".

La méthodologie employée dans les essais effectués dans ces applications de l'invention a été la suivante :

. pour <u>Neisseria gonorrhoeae</u> :

Les germes employés proviennent d'isolements hospitaliers.

- gamme de dilution des substances à tester : Solvant : $H_2O$ bidistillée stérile
Gamme de raison géométrique 2
Doses : de 2000 à 1, 56 (et moins) µg/ml
    2 ml de chacune des dilutions sont mélangés à 18 ml de milieu de culture solide préférentiel : milieu gélose d'isolement des gonocoques (Institut Pasteur) enrichi de supplément G :
Formule :
.   Sérum de Poulain 165 ml
.   Extrait de levure 100 ml
.   Extrait globulaire 235 ml
.   Glucose 0,65 ml
.   $H_2O$ distillée q.s.p. 500 ml
- milieu de culture des gonocoques : 2000 ml de milieu pasteur + 500 ml de supplément G

Ces milieux de culture associés aux dilutions des substances active représentent les moyens d'investigation de l'activité bactéricide. Les concentrations finales des substances seront de l'ordre de 800 à 0,156 µg/ml dans les boîtes de Pétri. Les suspensions utilisées (dilution dans l'eau physiologique d'une culture de 24 heures en milieu liquide -bouillon nutritif) sont placées dans des puits creusés dans un socle synthétique sous hotte à flux laminaire. Dans chacun des puits préparés, on a étudié une souche (ensemenceur multiple). Chaque boîte étudiée est examinée au bout de 24 et 48 heures de culture (Etuve 37° C).

- détermination de la CMI : les souches de gonocoque sont étalées sur des plaques (dilution sur plaque) dont certaines sont préparées à titre de témoins et de contrôle, sans substance à tester.

5 µl de 10 colonies formatrices/ml déposées sur les plaques de gélose complétée ou non avec les substances à tester (culture de 18 heures de gonocoques en suspension dans une solution de Sorensen).

On pratique une incubation de 48 h à 36° C, puis on observe sur les plaques la croissance ou non des gonocoques.

. pour Candida albicans :

La méthodologie est sensiblement la même que pour neisseria gonorrhoeae, mais avec les différences suivantes :
- Gamme de dilution dans l'eau bidistillée stérile de 2000 à 1,56 μg/ml
  0,5 ml de chaque dilution est additionné de 4,5 ml de milieu liquide de Roiron.
- Les tubes sont ensemencés avec des cultures de candida (24 heures/milieu Roiron) ou avec le témoin (milieu Roiron). L'incubation des tubes a lieu à l'étuve à 37° C pendant 24 et 48 heures. A t = 24 heures et t = 48 heures, observation d'une goutte entre lame et lamelle sous microscope.
- Expression des résultats par comptage des levures, et détermination d'une CMI fongicide. Sur les tubes témoins, détermination du pourcentage de résistants.

Pour Trichomonas Vaginalis :

Même méthodologie que pour Candida Albicans.

. Pour Chlamydia :

- Inoculum : il est représenté par les cellules-hôte des chlamydia ou cellules Mac Coy. Elles sont conservées à -80°. La concentration cellulaire sera pour les essais de 2 à 2,5 x 10 cellules/ml. La couche cellulaire est préparée dans un milieu de culture complet.
- Les substances à tester sont diluées en gamme croissante de raison géométrique 2 dans l'eau bidistillée stérile.
- La méthode employée est celle du Professeur F. CATALAN, P. SEDNAOUI, A. MILOVANOVIC et Coll., Institut A. FOURNIER, PARIS.

1 ml de dilution des substances à tester est mélangée à 1 ml de la suspension cellulaire (cellule Mac Coy), puis incubé 1 heure et 24 heures à 37° C à l'étuve. 0,2 ml de ce mélange est alors placé dans les plaques des puits. Les plaques sont centrifugées (1 heure à 2000 tours/mn) et incubées 1 heure à 37° C. Le milieu de culture est alors remplacé par 0,2 ml de milieu neuf contenant 0,5 mg/l de cycloheximide et les plaques sont alors incubées 48 heures à 37° C. La couche cellulaire est ensuite fixée au Méthanol et surtout colorée avec un anticorps monoclonal et conjugué à la F.I.T.C. (isothiocyanate de fluorescéine). A partir de là, on observe au microscope inversé à épifluorescence les inclusions présentes dans les cellules de Mac Coy.

On a donc dans un premier temps étudié la toxicité des substances à tester dont on a tenu compte pour respecter l'intégrité totale de la cellule hôte.

. Pour Pseudomonas aeruginosa :

- Préparation d'une solution dense de deux souches.
- Suspension en solution isotonique de NaCl (0,85 % en poids).
- Vérification que les suspensions renferment le même nombre de bactéries : techniques usuelles en milieu gélosé par ensemencement en stries (anse de 1 μl) des dilutions successives de la suspension dense de départ (raison géométrique 10). Incubation 18 h à 37° C.
- Préparation des dilutions des substances à tester en solution dans l'eau bidistillée stérile. Gamme de dilution de raison géométrique 2 (de 2000 à 0,015 μg/ml par exemple).
- Mélange de la suspension bactérienne Pseudomonas aeruginosa (de même origine que les normes AFNOR), avec une concentration constante (= $4 \times 10^7$ bactéries/ml) et de la dilution des substances dans un ordre décroissant (2000 à 0,015 μg/ml).
- Incubation ou temps de contact : 1 heure, 24 heures et 48 heures, à l'étuve à 37° C.
  Les essais ont été doublés pour chaque dilution de substance.
- Observation du nombre de survivants enregistré par rapport au nombre moyen de souches témoin cultivées en même temps que les essais.
- Observation après 1 heure, 24 heures et 48 heures.

. Pour Tréponèmes, Gardnerella, Bacille de Ducrey, Streptocoques, et Staphylococcus aureus :

La méthodologie utilisée est celle décrite par le Professeur F. CATALAN, P. SEDNAOUI, A. MILOVANOVIC et Coll., Institut A. FOURNIER.

ESSAI COMPARATIF N° 7 :

Avec du chlorure de benzalkonium seul et du nonoxynol 9 seul, les résultats suivants ont été obtenus :

| SOUCHES | CONCENTRATION MINIMALE INHIBITRICE (CMI) |
|---|---|
| | Chlorure de benzalkonium seul |
| Gonocoque | 1,15 mg/l |
| Tréponèmes | 70 mg/l |
| Trichomonas | 1,3 mg/l |
| Candidas Albicans | 50 mg/l |
| Chlamydia | 100 mg/l |
| Gardnerella | 50 mg/l |

| | |
|---|---|
| Bacille de Ducrey | 75 mg/l |
| Streptocoque | 15 mg/l |
| Pseudomonas Aeruginosa | 31,25 mg/l |
| Staphylocoque Aureus | 1,56 mg/l |

| SOUCHES | CONCENTRATION MINIMALE INHIBITRICE (CMI) |
|---|---|
| | Nonoxynol 9 seul |
| Tréponèmes | 75 mg/l |
| Pseudomonas aeruginosa | 50 mg/l |
| Staphylocoque aureus | 4 mg/l |
| Streptocoque | 20 mg/l |

ESSAI N° 8 :

Le même essai que précédemment, réalisé en présence d'anion fluorure $F^-$ a mené aux résultats suivants :

| SOUCHES | CONCENTRATION MINIMALE INHIBITRICE (CMI) |
|---|---|
| | Chlorure de benzalkonium + F⁻ 1 microgramme / ml |
| Gonocoque | 0,60 mg/l |
| Tréponèmes | 56 mg/l |
| Trichomonas | 0,9 mg/l |
| Candidas Albicans | 35 mg/l |
| Chlamydia | 85 mg/l |
| Gardnerella | 41 mg/l |
| Bacille de Ducrey | 62 mg/l |
| Streptocoque | 9 mg/l |
| Pseudomonas Aeruginosa | 18 mg/l |
| Staphylocoque Aureus | 1,1 mg/l |

| SOUCHES | CONCENTRATION MINIMALE INHIBITRICE (CMI) |
|---|---|
| | Nonoxynol 9 + F⁻ 1 microgramme / ml |
| Tréponèmes | 60 mg/l |
| Pseudomonas aeruginosa | 35 mg/l |
| Staphylocoque aureus | 2,5 mg/l |
| Streptocoque | 15 mg/l |

ESSAI COMPARATIF N° 9 :

Cet essai a été mené avec une substance comportant du chlorure de benzalkonium, sans principe activateur, mais en présence de protéines sériques. Il a mené aux résultats suivants :

| SOUCHES | CONCENTRATIONS EN PROTEINES SERIQUES | CONCENTRATION MINIMALE INHIBITRICE (CMI) | |
|---|---|---|---|
| | | Chlorure de benzalkonium seul | |
| Streptococcus Faecalis | O | 15 | mg/ml |
| | 30 mg/ml | 15 | mg/ml |
| | 60 mg/ml | 16 | mg/ml |
| | 90 mg/ml | 68 | mg/ml |
| Neisseria Gonorrhoeae | O | 1,5 | mg/ml |
| | 30 mg/ml | 1,5 | mg/ml |
| | 60 mg/ml | 1,5 | mg/ml |
| | 90 mg/ml | 9 | mg/ml |

Cet essai montre l'effet néfaste bien connu des protéines sériques sur l'efficacité du chlorure de benzalkonium.

ESSAI N° 10 :

Cet essai a été mené de façon identique à l'essai N° 9, mais en présence d'anion fluorure F⁻ dans la composition. Les résultats suivants ont été obtenus :

| SOUCHES | CONCENTRATIONS EN PROTEINES SERIQUES | CONCENTRATION MINIMALE INHIBITRICE (CMI) |
|---------|--------------------------------------|-------------------------------------------|
|         |                                      | Chlorure de benzalkonium + $F^-$ 1 microgramme / ml |
| Streptococcus Faecalis | 0 | 9 mg/ml |
|         | 30 mg/ml | 9 mg/ml |
|         | 60 mg/ml | 9 mg/ml |
|         | 90 mg/ml | 21 mg/ml |
| Neisseria Gonorrhoeae | 0 | 0,6 mg/ml |
|         | 30 mg/ml | 0,6 mg/ml |
|         | 60 mg/ml | 0,6 mg/ml |
|         | 90 mg/ml | 1,2 mg/ml |

Cet essai montre que l'anion fluorure $F^-$ permet de diminuer très favorablement l'effet néfaste des protéines sériques.

ESSAI COMPARATIF N° 11 :

Cet essai a été mené sur les formes galéniques in vitro (de façon similaire aux essais 5 et 6), à partir de produits contenant du chlorure de benzalkonium comme principe actif et sans principe activateur. Les résultats suivants ont été obtenus :

| SOUCHES | FORME GALENIQUE | CONCENTRATION MINIMALE INHIBITRICE (CMI) |
|---------|-----------------|-------------------------------------------|
|         |                 | Chlorure de benzalkonium seul |
| Gonocoque | ovule | 23,65 mg/l |
|         | comprimé | 15,62 mg/l |
| Trichomonas | ovule | 11,80 mg/l |
|         | comprimé | 15,60 mg/l |

Les concentrations sont celles présentes dans le liquide obtenu après simulation, utilisé pour le test.

ESSAI N° 12 :

EP 0 253 037 B1

Cet essai a été mené dans les mêmes conditions que l'essai. comparatif N° 11, à partir de produits contenant 0,5 % (en poids) d'anion fluorure $F^-$. [es résultats suivants ont été obtenus :

| SOUCHES | FORME GALENIQUE | CONCENTRATION MINIMALE INHIBITRICE (CMI) |
|---|---|---|
| | | Chlorure de benzalkonium + $F^-$ à 0,5 % |
| Gonocoque | ovule comprimé | 5 mg /l<br>3 mg /l |
| Trichomonas | ovule comprimé | 5 mg /l<br>3 mg /l |

III) APPLICATION DE L'INVENTION DANS LE DOMAINE DE LA DESINFECTION :

Ce domaine concerne le traitement des sols, surfaces, instruments ... par des produits bactéricides de contact.

Les modes de réalisation préférentiels de l'invention pour cette application sont les suivants :

22

| USAGES | CHLORURE DE BENZALKONIUM | Anion F⁻ | EXCIPIENTS |
|---|---|---|---|
| Mains<br><br>Epiderme | 0,1 % | 0,25 % | Eau purifiée ou alcool q.s.p. 100 % |
| Instruments à stériliser à désinfecter | 1,0 % | 0,50 % | Eau purifiée<br><br>q.s.p. 100 % |
| Textiles | 0,05 % | 0,025 % | Eau purifiée<br><br>q.s.p. 100 % |
| Instruments<br><br>type thermomètre | 1,0 % | 0,50 % | Ethanol 10 %<br>Eau purifiée<br>q.s.p. 100 % |
| Lavages des surfaces (chambres, planchers, sols) | 0,1 % | 0,25 % | Eau purifiée<br><br>q.s.p. 100 % |

Les essais ont été menés suivant la norme AFNOR NFT 72-150 mars 81 avec du chlorure de benzalkonium, puis avec du nonoxynol 9 comme principe actif. Le neutralisant utilisé a été le suivant : 3 % de Tween® 80 (V/V) et 0,3 % de lécithine (M/V). Le pH du milieu était de 7,2.

ESSAI COMPARATIF N° 13 :

Cet essai a été mené sans principe activateur.

| SOUCHES | CONCENTRATION MINIMALE INHIBITRICE (CMI) |
| --- | --- |
| | Chlorure de benzalkonium seul |
| Pseudomonas aeruginosa CNCM A 22 | 31,25 mg/litre |
| Escherichia coli CNCM 54 127 | 6,57 mg/l |
| Staphylococcus Aureus souche Oxford CNCM 53 154 | 1,56 mg/l |
| Streptococcus faecalis CNCM 5 855 | 4 mg/l |
| Mycobacterium smegmatis CNCM 7 326 | 30 mg/l |

| SOUCHES | CONCENTRATION MINIMALE INHIBITRICE (CMI) |
| --- | --- |
| | Nonoxynol 9 seul |
| Pseudomonas aeruginosa CNCM A 22 | 50 mg/litre |
| Escherichia coli CNCM 54 127 | 8 mg/l |
| Staphylococcus Aureus souche Oxford CNCM 53 154 | 4 mg/l |
| Streptococcus faecalis CNCM 5 855 | 7 mg/l |
| Mycobacterium smegmatis CNCM 7 326 | 65 mg/l |

ESSAI N° 14 :

Cet essai est identique au précédent, mais en présence d'anion fluorure $F^-$ à titre de principe activateur.

| SOUCHES | CONCENTRATION MINIMALE INHIBITRICE (CMI) | |
|---|---|---|
| | Chlorure de benzalkonium + F⁻ 1 microgramme / ml | |
| Pseudomonas aeruginosa CNCM A 22 | 18 | mg/litre |
| Escherichia coli CNCM 54 127 | 3 | mg/l |
| Staphylococcus Aureus souche Oxford CNCM 53 154 | 1,1 | mg/l |
| Streptococcus faecalis CNCM 5 855 | 3,6 | mg/l |
| Mycobacterium smegmatis CNCM 7 326 | 26 | mg/l |

| SOUCHES | CONCENTRATION MINIMALE INHIBITRICE (CMI) | |
|---|---|---|
| | Nonoxynol 9 seul + F⁻ 1 microgramme / ml | |
| Pseudomonas aeruginosa CNCM A 22 | 35 | mg/litre |
| Escherichia coli CNCM 54 127 | 6,5 | mg/l |
| Staphylococcus Aureus souche Oxford CNCM 53 154 | 2,5 | mg/l |
| Streptococcus faecalis CNCM 5 855 | 5,5 | mg/l |
| Mycobacterium smegmatis CNCM 7 326 | 50 | mg/l |

IV) APPLICATION DE L'INVENTION EN COSMETOLOGIE :

Les modes de réalisation préférentiels de l'invention en cosmétologie sont décrits ci-après.

Les formes galéniques suivantes peuvent être présentées en cosmétologie : crèmes, laits, pommades, solutions, bains moussants, savons synthétiques, shampooings, lotion intime, lotion désinfectante.

Les formules des excipients sont les mêmes que pour les présentations pharmaceutiques mais les concentrations en chlorure de benzalkonium et en anion F⁻ seront différentes.

Ces concentrations seront donc les suivantes pour tous les produits : Chlorure de benzalkonium : 0,2 %, anion F⁻ : 0,1 %.

Peuvent également entrer dans ces formulations des principes actifs chimiques ou d'origine naturelle aux concentrations et doses autorisées en cosmétologie.

Les crèmes et laits peuvent être en phase continue aqueuse (émulsion huile/eau ou eau/huile) ou pâteuse à chaud se diluant dans l'eau.

Les différents excipients cités à titre d'exemple correspondent à titre indicatif non limitatif aux produits suivants :

. Humectants : glycérol, propylène glycol, diéthylène glycol, sorbitol, polyoxyéthylène glycol.

. Emulseurs : stéarate de sodium, cire d'abeilles, ester de sorbitol, ester de polyoxyéthylène glycol, alcool gras, triethanolamine lanoline, tween, stéarate de glycol et polyglycols.

. Stabilisateurs : stéarate de glycol, alcool cétylique alginate, pectine, gomme, esters gras de polyols, esters de cellulose solubles.

. Antioxygène : acide tartrique, citrique et ascorbique.

. Antiseptique : acide borique, acide benzoique, acide parabenzoique et leurs esters méthyliques ou propyliques, sodés ou non.

. PH : toutes ces formulations sont particulièrement efficaces à des pH compris entre 4,5 et 6, 5. Pour obtenir cette fourchette on utilise principalement l'acide citrique.

## Revendications

1. Application d'au moins un principe actif inhibiteur ou destructeur d'au moins un être vivant unicellulaire ou virus et d'un principe activateur dans des proportions appropriées pour inhiber ou détruire au moins un enzyme associé audit être vivant unicellulaire ou virus pour obtenir un produit ou un médicament destiné à inhiber ou détruire cet être vivant unicellulaire ou virus, à l'exception du cas de l'association de l'acide benzoïque, du phénol et d'un fluorure alcalin en solution aqueuse ou hydroalcoolique pour obtenir un produit destiné à inhiber ou détruire le virus de l'herpès.

2. Application selon la revendication 1 dans laquelle le principe activateur est un composé fluoré émetteur de fluor ionique.

3. Application selon la revendication 2, dans laquelle le composé fluoré est un dérivé métallique du fluor.

4. Application selon la revendication 3, dans laquelle le composé fluoré est choisi parmi le fluorure de sodium, le fluorure de calcium, le fluorure de potassium, le fluorure d'aluminium, le fluorure d'étain, le fluorure d'ammonium et le monofluorophosphate de sodium.

5. Application selon la revendication 1 dans laquelle le principe activateur est l'anion fluorure F⁻.

6. Application selon l'une quelconque des revendications 1 à 5, dans laquelle un principe actif est un composé tensio-actif surfactif.

7. Application selon la revendication 6, dans laquelle le principe actif est cationique.

8. Application selon la revendication 7, dans laquelle le principe actif est un ammonium quaternaire.

9. Application selon l'une quelconque des revendications 6 à 8 dans laquelle le principe actif est le chlorure de benzalkonium.

10. Application selon la revendication 6 dans laquelle un principe actif est un composé tensio-actif surfactif anionique.

11. Application selon la revendication 6 dans laquelle un principe actif est un composé tensio-actif surfactif amphotère.

12. Application selon la revendication 6 dans laquelle un principe actif est un composé tensio-actif surfactif non-ionique.

13. Application selon l'une quelconque des revendications 6 à 12 dans laquelle un principe actif est choisi parmi un méthyltauride d'oxyde sel sodique, un dérivé d'amidoéthylglycinate sur acide gras, un nonoxynol, le nitrate de phénylmercure, le para-menthanylphénylpolyoxyéthylène éther, ou un sel trisodique de l'éther sulfurique de polysaccharide.

14. Application selon l'une quelconque des revendications 1 à 13 pour obtenir un produit ou un médicament destiné à inhiber ou détruire un protozoaire, ou un microbe, ou une bactérie, ou un gamète, ou un champignon, ou un virus.

15. Application selon l'une quelconque des revendications 1 à 14 pour obtenir un produit ou un médicament destiné à inhiber ou détruire au moins un être vivant unicellulaire ou virus selectionné parmi les gamètes, coques à Gram positif, coques à Gram négatif, bacilles à Gram positif, bacilles à Gram négatif, bacilles acido-alcoolo-résistants, bactéries, protozoaires flagellés, levures, virus ou rétro-virus.

16. Application selon l'une quelconque des revendications 1 à 15 d'au moins un principe actif inhibiteur ou destructeur de gamètes et d'un principe activateur dans des proportions appropriées pour inhiber ou détruire au moins un enzyme associé audit gamète pour obtenir un produit contraceptif local.

17. Application selon la revendication 16 de chlorure de benzalkonium et d'un composé fluoré émetteur de fluor ionique dans des proportions appropriées pour obtenir un produit sous forme d'ovule, de crème, de lait, d'onguent, de pommade, de gelée ou de film soluble contenant 1,20 % en poids de chlorure de benzalkonium et 0,50 % en poids d'anion F⁻ , ou un produit sous forme de comprimé contenant 25 mg par comprimé de chlorure de benzalkonium et 10 mg par comprimé d'anion F⁻ , ou un produit sous forme de savon synthétique contenant 2 % en poids de chlorure de benzalkonium et 1 % en poids d'anion F⁻, ou un produit sous forme de solution contenant 0,50 % en poids de chlorure de benzalkonium et 0,25 % en poids d'anion F.⁻.

18. Application selon la revendication 16 dans laquelle un principe actif est un composé fluoré susceptible d'émettre du fluor ionique.

19. Application selon l'une quelconque des revendications 1 à 15 d'au moins un principe actif inhibiteur ou destructeur d'au moins un être vivant unicellulaire responsable de maladies sexuellement transmissibles et d'un principe activateur dans des proportions appropriées pour inhiber ou détruire au moins un enzyme associé audit être vivant unicellulaire pour obtenir un médicament destiné à lutter contre les maladies sexuellement transmissibles.

20. Application selon la revendication 19 de chlorure de benzalkonium et d'un composé fluoré émetteur de fluor ionique dans des proportions appropriées pour obtenir un produit sous forme d'ovule, de crème, de lait, d'onguent, de pommade, de gelée ou de film soluble contenant 1,20 % en poids de chlorure de benzalkonium et 0,50 % en poids d'anion F⁻ , ou un produit sous forme de comprimé contenant 25 mg par comprimé de chlorure de benzalkonium et 10 mg par comprimé d'anion F⁻, ou un produit sous forme de savon synthétique contenant 2 % en poids de chlorure de benzalkonium et 1 % en poids d'anion F⁻, ou un produit sous forme de solution contenant 0,50 % en poids de chlorure de benzalkonium et 0,25 % en poids d'anion F.⁻.

21. Application selon la revendication 19 dans laquelle un principe actif est un composé fluoré susceptible d'émettre du fluor ionique.

22. Application selon l'une quelconque des revendications 1 à 15 d'au moins un principe actif ayant une action antiobiotique vis-à-vis d'au moins un être vivant unicellulaire et d'un principe activateur dans des proportions appropriées pour inhiber ou détruire au moins un enzyme associé audit être vivant unicellulaire pour obtenir un médicament pour lutter par antibiothérapie contre les maladies dont ledit être vivant unicellulaire est responsable.

23. Application selon la revendication 22 de chlorure de benzalkonium ou d'érythromycine base ou de néomycine base ou de bacitracine ou d'oxytétracycline chlorhydrate ou d'auréomycine chlorhydrate ou de soframycine sulfate ou d'auréomycine, ou d'oxytétracycline et d'un composé fluoré susceptible d'émettre du fluor ionique pour obtenir un médicament sous forme de crème, ou de lait, ou d'onguent ou de pommade contenant 1,20 % en poids de chlorure de benzalkonium et 0,50 % en poids d'anion F⁻, ou un médicament sous forme de savon synthétique contenant 2 % en poids de chlorure de benzalkonium et 1 % en poids d'anion F⁻ , ou un médicament sous forme de solution contenant 0,50 % en poids de chlorure de benzalkonium et 0,25 % en poids d'anion F⁻, ou un médicament sous forme de solution ou de gel contenant 4 % en poids d'érythromycine base et 0,5 % en poids d'anion F⁻, ou un médicament sous forme de pommade contenant 0,35 % en poids de néomycine base et 0,50 % d'anion F⁻, ou 50.000 UI % et 0,50 % d'anion F⁻, ou 3 % en poids d'oxytétracycline chlorhydrate et 0,50 % en poids d'anion F⁻, ou 3 % en poids d'auréomycine chlorhydrate et 0,50 % en poids d'anion F⁻, ou un médicament sous forme de crème contenant 2,5 % en poids de soframycine sulfate et 0,50 % en poids d'anion F⁻, ou un médicament sous forme de pommade ophtalmique contenant 1 % en poids d'auréomycine et 0,50 % en poids d'anion F⁻, ou 0,50 % en poids d'oxytétracycline et 0,25 % en poids d'anion F⁻, ou un médicament sous forme de solution nasale contenant 1,25 % en poids de de soframycine sulfate et 0,50 % en poids d'anion F⁻.

24. Application selon la revendication 22 de chlorure de benzalkonium ou d'érythromycine base ou de néomycine base ou de bacitracine ou d'oxytétracycline chlorhydrate ou d'auréomycine chlorydrate ou de soframycine sulfate ou d'auréomycine, ou d'oxytatracycline et d'un composé fluoré susceptible d'émettre un fluor ionique pour obtenir un médicament sous forme de solution ou de gel contenant 4 % en poids d'érythromycine base, 0,5 % en poids d'anion F⁻ et 0,10 % de chlorure de benzalkonium, ou un médicament sous forme de pommade contenant 0,35 % en poids de néomycine base, 0,50 % d'anion F⁻, et 0,10 % de chlorure de benzalkonium, ou 50.000 UI %, 0,50 % d'anion F⁻ et 0,10 % de chlorure de benzalkonium, ou 3 % en poids d'oxytrétracycline chlorydrate, 0,50 % en poids d'anion F⁻ et 0,10 % de chlorure de benzalkonium, ou 3 % en poids d'auréomycine chlorhydrate, 0,50 % en poids d'anion F- et 0,10 % de chlorure de benzalkonium, ou un médicament sous forme de crème contenant 2,5 % en poids de soframycine sulfate, 0,50 % en poids d'anion F⁻ et 0,10 % de chlorure de benzalkonium ou un médicament sous forme de pommade ophtalmique contenant 1 % en poids d'auréomycine, 0,50 % en poids d'anion F⁻ et 0,10 % de chlorure de benzalkonium ou 0,50 % en poids d'oxytétracycline, 0,25 % en poids d'anion F⁻ et 0,10 % de chlorure de benzalkonium, ou un médicament sous forme de solution nasale contenant 1,25 % en poids de soframycine sulfate, 0,50 % en poids d'anion F⁻ et 0,10 % de chlorure de benzalkonium.

25. Application selon l'une quelconque des revendication 1 à 15, d'au moins un principe actif inhibiteur ou destructeur d'au moins un champignon et d'un principe actif dans des proportions appropriées pour inhiber ou détruire au moins un enzyme associé audit champignon pour obtenir un produit antifongique.

26. Application d'au moins un principe actif inhibiteur ou destructeur d'au moins un protozoaire et d'un principe activateur dans des proportions appropriées pour inhiber ou détruire au moins un enzyme associé audit protozoaire pour obtenir un produit anti-protozoaire.

27. Application d'au moins un principe actif inhibiteur ou destructeur d'au moins une bactérie et d'un principe activateur dans des proportions appropriées pour inhiber ou détruire au moins un enzyme associé à ladite bactérie pour obtenir un produit ou un médicament bactéricide.

28. Application selon la revendication 27 de chlorure de benzalkonium et d'un composé fluoré émetteur de fluor ionique pour obtenir un produit bactéricide de contact contenant 0,1% de chlorure de benzalkonium et 0,25 % d'anion F⁻, ou 1 % de chlorure de benzalkonium et 0,50 % d'anion F⁻, ou 0,05 % de chlorure de benzalkonium et 0,025 % d'anion F⁻.

29. Application selon l'une quelconque des revendications 1 à 15, d'au moins un principe actif inhibiteur ou destructeur d'au moins un virus ou rétro-virus et d'un principe activateur dans des proportions appropriées pour inhiber ou détruire au moins un enzyme associé audit virus ou rétro-virus pour obtenir un produit ou un médicament antiviral.

**Claims**

1. Application of at least one active principle, which inhibits or destroys at least one unicellular living being or virus and of an activating principle in proportions suitable for inhibiting or destroying at least one enzyme associated with the said unicellular living being or virus to obtain a product or a medicament for inhibiting or destroying this unicellular living being or virus, with the exception of the case of the association of benzoic acid, phenol and an alkaline fluoride in aqueous solution or hydroalcoholic solution, to obtain a product for inhibiting or destroying the herpes virus.

2. Application according to Claim 1, in which the activating principle is a fluorine compound emitting ionic fluorine.

3. Application according to Claim 2, in which the fluorine compound is a metal derivative of fluorine.

4. Application according to Claim 3, in which the fluorine compound is selected from the group comprising sodium fluoride, calcium, fluoride, potassium fluoride, aluminium fluoride, tin fluoride, ammonium fluoride and sodium monofluorophosphate.

5. Application according to Claim 1, in which the activating principle is the fluoride anion $F^-$.

6. Application according to any one of Claims 1 to 5, in which an active principle is a surfactant surface-active compound.

7. Application according to Claim 6, in which the active principle is cationic.

8. Application according to Claim 7, in which the active principle is a quaternary ammonium.

9. Application according to any one of Claims 6 to 8, in which the active principle is benzalkonium chloride.

10. Application according to Claim 6, in which an active principle is an anionic surfactant surface-active compound.

11. Application according to Claim 6, in which an active principle is an amphoteric surfactant surface-active compound.

12. Application according to Claim 6, in which an active principle is a non-ionic surfactant surface-active compound.

13. Application according to any one of Claims 6 to 12, in which an active principle is selected from the group comprising a sodium salt oxide methyltauride, a derivative of aminocthylglycinate of fatty acid, a nonoxynol, phenylmercury nitrate, para-menthanylphenylpolyoxyethylene ether, or a triosodium salt of polysaccharide sulphuric ether.

14. Application according to any one of Claims 1 to 13, to obtain a product or a medicament for inhibiting or destroying a protozoan, or a microbe, or a bacterium, or a gamete, or a fungus, or a virus.

15. Application according to any one of Claims 1 to 14, to obtain a product or a medicament for inhibiting or destroying at least one unicellular living beirg or a virus selected from the group comprising gametes, Gram-positive cocci, Gram-negative cocci, Gram-Positive bacilli, Gram-negative bacilli, bacilli resistant to acid alcohols, bacteria, flagellated protozoa, yeasts, viruses or retro-viruses.

16. Application according to any one of Claims 1 to 15 of at least one active principle which inhibits or destroys gametes and of an activating principle in proportions suitable for inhibiting or destroying at least one enzyme associated with the said gamete to obtain a local contraceptive product.

17. Application according to Claim 16 of benzalkonium chloride and of a fluorine compound emitting ionic fluorine, in proportions suitable for obtaining a product in the form of a pessary, cream, lotion, unguent, ointment, gel or soluble film containing 1.20% by weight of benzalkonium chloride and 0.50% by weight of the anion $F^-$, or a product in the form of a tablet containing 25 mg per tablet of benzalkonium chloride and 10 mg per tablet of the anion $F^-$, or a product in the form of a synthetic soap containing 2% by weight of benzalkonium chloride and 1% by weight of the anion $F^-$, or a product in the form of a solution containing 0.50% by weight of benzalkonium chloride and 0.25 % by weight of the anion $F^-$.

18. Application according to Claim 16, in which an active principle is a fluorine compound capable of emitting ionic fluorine.

19. Application according to any one of Claims 1 to 15 of at least one active principle which inhibits or destroys at least one unicellular living being responsible for sexually transmitted disesaes and an activating principle in proportions suitable for inhibiting or destroying at least one enzyme associated with the said unicellular living being to obtain a medicament intended to combat sexually transmitted diseases.

20. Application according to Claim 19 of benzalkonium chloride and a fluorine compound emitting ionic fluorine in proportions suitable for obtaining a product in the form of a pessary, cream, lotion, unguent, ointment, gel or soluble film, containing 1.20% by weight of benzalkonium chloride and 0.50% by weight of the anion $F^-$, or a product in the form of a tablet containing 25 mg per tablet of benzalkonium chloride and 10 mg per tablet of the anion $F^-$, or a product in the form of a synthetic soap containing 2% by weight of benzalkonium chloride and 1% by weight of the anion $F^-$, or a product in the form of a solution containing O.50 % by weight of benzalkonium chloride and 0.25% by weight of the anion $F^-$.

21. Application according to Claim 19, in which an active principle is a fluorine compound capable of emitting ionic fluorine.

22. Application according to any one of Claims 1 to 15 of at least one active principle having an antibiotic action towards at least one unicellular living being and of an activating principle in proportions suitable for inhibiting or destroying at least one enzyme associated with the said unicellular living being to obtain a medicament to combat by antibiotic therapy the diseases for which the unicellular living being is responsible.

23. Application according to Claim 22 of benzalkonium chloride or erythromycin base or neomycin base or bacitracin or oxytetracycline hydrochloride or aureomycin hydrochloride or soframycin sulphate or aureomycin or oxytetracycline and a fluorine compound capable of emitting ionic fluorine to obtain a medicament in the form of cream or lotion, or unguent or ointment containing 1.20% by weight of benzalkonium chloride and 0.50% by weight of the anion $F^-$, or a medicament in the form of a synthetic soap containing 2% by weight of benzalkonium chloride and 1% by weight of the anion $F^-$, or a medicament in the form of a solution containing 0.50% by weight of benzalkonium chloride and 0.25% by weight of the anion $F^-$, or a medicament in the form of s solution or a gel containing 4% by weight of erythromycin base and 0.5% by weight of the anion $F^-$, or a medicament in the form of an ointment containing 0.35% by weight of neomycin base and 0.50% by weight of the anion $F^-$, or 50,000 international untits % and 0.50% of the anion $F^-$, or 3% by weight of oxytetracycline hydrochloride and 0.50% by weight of the anion $F^-$, or 3% by weight of aureomycin hydrochloride and 0.50% by weight of the anion $F^-$, or a medicament in the form of a cream containing 2.5% by weight of soframycin sulphate and 0.50% by weight of the anion $F^-$, or a medicament in the form of an ophthalmic ointment containing 1% by weight of aureomycin and 0.50% by weight of the anion $F^-$, or 0.50% by weight of oxytetracycline and 0.25% by weight of the anion $F^-$, or a medicament in the form of a nasal solution containing 1.25% by weight of soframycin sulphate and 0.50% by weight of the anion $F^-$.

24. Application according to Claim 22 of benzalkonium chloride or erythromycin base or neomycin base or bacitracin or oxytetracycline hydrochloride or aureomycin hydrochloride or soframycin sulphate or aureomycin, or oxytetracycline and a fluorine compound capable of emitting ionic fluorine to obtain a medicament in the form of a solution or a gel containing 4% by weight of erythromycin base, 0.5% by weight of the anion $F^-$ and 0.10% of benzalkonium chloride, or a medicament in the form of an ointment containing 0.35% by weight of neomycin base, 0.50% of the anion $F^-$, and 0.10% of benzalkonium chloride or 50,000 international units %, 0.50% of the anion $F^-$ and 0.10% of benzalkonium chloride, or 3% by weight of oxytetracycline hydrochloride, 0.50% by weight of the anion $F^-$ and 0.10% of benzalkonium chloride, or 3% by weight of aureomycin hydrochloride, 0.50% by weight of the anion $F^-$ 0.10% of benzalkonium chloride, or a medicament in the form of cream containing 2.5% by weight of soframycin sulphate, 0.50% by weight of the anion $F^-$ and 0.10% of benzalkonium chloride or a medicament in the form of an ophthalmic ointment containing 1% by weight of aureomycin, 0.50% by weight of the anion $F^-$ and 0.10% of benzalkonium chloride or 0.50% by weight

30

of oxytetracycline, 0.25% by weight of the anion F⁻ and 0.10% of benzalkonium chloride, or a medicament in the form of a nasal solution containing 1.25% by weight of soframycin sulphate, 0.50% by weight of the anion F⁻ and 0.10% of benzalkonium chloride.

25. Application according to any one of Claims 1 to 15, of at least one active principle which inhibits or destroys at least one fungus and as an active principle in proportions suitable for inhibiting or destroying at least one enzyme associated with the said fungus to obtain an antifungal product.

26. Application of at least one active principle which inhibits or destroys at least one protozoan of an activating principle in proportions suitable for inhibiting or destroying at least one enzyme associated with the said protozoan to obtain an anti-protozoic product.

27. Application of at least one active principle which inhibits or destroys at least one bacterium and of an activating principle in proportions suitable for inhibiting or destroying at least one enzyme associated with the said bacterium to obtain a bactericidal medicament or product.

28. Application according to Claim 27, of benzalkonium chloride and of fluorine compound emitting ionic fluorine to obtain a batericidal contact product containing 0.1% of benzalkonium chloride and 0.25% of the anion F⁻, or 1% of benzalkonium chloride and 0.50% of the anion F⁻, or 0.05% of benzalkonium chloride and 0.025% of the anion F⁻.

29. Application according to any one of Claims 1 to 15, of at least one active principle which inhibits or destroys at least one virus or retrovirus and of an activating principle in proportions suitable for inhibiting or destroying at least one enzyme associated with the said virus or retrovirus to obtain an antiviral medicament or product.

**Ansprüche**

1. Anwendung von mindestens einem Wirkprinzip, das mindestens ein einzelliges Lebewesen oder Virus inhibiert oder vernichtet, und von einem Giftungs-Prinzip in Mengenverhältnissen, die dazu geeignet sind, mindestens ein mit dem besagten einzelligen Lebewesen oder Virus assoziiertes Enzym zu hemmen oder zu zerstören, um ein Produkt oder ein Medikament zu gewinnen, das zur Hemmung oder Vernichtung dieses einzelligen Lebewesens oder Virus bestimmt ist, mit Ausnahme des Falles der Assoziation von Benzoesäure, Phenol und eines alkalischen Fluorids in wässeriger oder wässerig-alkoholischer Lösung zur Gewinnung eines Produkts, das dazu bestimmt ist, das Herpes-Virus zu hemmen oder zu vernichten.

2. Anwendung nach Anspruch 1, in der das Giftungs-Prinzip eine fluorhaltige Verbindung ist, die Fluorionen freisetzt.

3. Anwendung nach Anspruch 2, in der die fluorhaltige Verbindung ein metallisches Fluorderivat ist.

4. Anwendung nach Anspruch 3, in der die fluorhaltige Verbindung unter Natriumfluorid, Calciumfluorid, Kaliumfluorid, Aluminiumfluorid, zinnfluorid, Ammoniumfluorid und Natriummonofluorophosphat gewählt wird.

5. Anwendung nach Anspruch 1, in der das Giftungs-Prinzip das Fluorid-Anion F⁻ ist.

6. Anwendung nach irgendeinem der Ansprüche 1 bis 5, in der ein Wirkprinzip eine grenzflächenaktive Verbindung ist.

7. Anwendung nach Anspruch 6, in der das Wirkprinzip kationisch ist.

8. Anwendung nach Anspruch 7, in der das Wirkprinzip ein quaternäres Ammonium ist.

9. Anwendung nach irgendeinem der Ansprüche 6 bis 8, in der das Wirkprinzip das Benzalkoniumchlorid ist.

10. Anwendung nach Anspruch 6, in der bin Wirkprinzip eine anionische grenzflächenaktive Verbindung ist.

11. Anwendung nach Anspruch 6, in der ein Wirkprinzip eine amphotere grenzflächenaktive Verbindung ist.

12. Anwendung nach Anspruch 6, in der ein Wirkprinzip eine nicht-ionische grenzflächenaktive Verbindung ist.

13. Anwendung nach irgendeinem der Ansprüche 6 bis 12, in der ein Wirkprinzip unter einem Methyltaurid - Natriumoxidsalz, einem Fettsäureamidoethylglycinat, einem Nonoxynol, dem Phenylquecksilbernitrat, dem Para-Menthanylphenylpolyoxyethylenether oder einem Trinatriumsalz des Polysaccharidschwefelethers gewählt wird.

14. Anwendung nach irgendeinem der Ansprüche 1 bis 13 zur Gewinnung eines Produkts oder eines Medikaments, das dazu bestimmt ist, ein Protozoon oder eine Mikrobe oder ein Bakterium oder einen Gameten oder einen Pilz oder ein Virus zu hemmen oder zu vernichten.

15. Anwendung nach irgendeinem der Ansprüche 1 bis 14 zur Gewinnung eines Produkts oder eines Medikaments, das dazu bestimmt ist, mindestens ein einzelliges Lebewesen oder Virus aus der Gruppe der Gameten, der Gram-positiven Kokken, Gramnegativen Kokken, Gram-positiven Bazillen, Gram-negativen Bazillen, der säure-alkohol-resistenten Bazillen, der Bakterien, der Flagellaten, der Hefen, der Viren oder Retroviren zu hemmen oder zu vernichten.

16. Anwendung nach irgendeinem der Ansprüche 1 bis 15 von mindestens einem wirkprinzip, das Gameten hemmt oder vernichtet, und von einem Giftungs-Prinzip in Mengenverhältnissen, die dazu geeignet sind, mindestens ein mit dem besagten Gameten assoziiertes Enzym zu hemmen oder zu zerstören, zur Gewinnung eines lokal-wirkenden empfängnisverhütenden Produkts (Lokalkontrazeptivums).

17. Anwendung nach Anspruch 16 von Benzalkoniumchlorid und von einer fluorhaltigen Verbindung, die Fluorionen freisetzt, in geeigneten Mengenverhältnissen zur Gewinnung eines Produkts in Form eines Suppositoriums, einer Creme, einer Milch, einer Salbe, einer Pomade, eines Gels oder eines löslichen Films, das 1,20 Gew.% Benzalkoniumchlorid und 0,50 Gew.% des $F^-$-Anions enthält, oder eines Produkts in Tablettenform, das pro Tablette 25 mg Benzalkoniumchlorid und 10 mg des $F^-$-Anions enthält, oder eines Produkts in Form einer synthetischen Seife, das 2 Gew.% Benzalkoniumchlorid und 1 Gew.% des $F^-$-Anions enthält, oder eines Produkts in Form einer Lösung, das 0,50 Gew.% Benzalkoniumchlorid und 0,25 Gew.% des $F^-$-Anions enthält.

18. Anwendung nach Anspruch 16, in der ein Wirkprinzip eine zur Freisetzung von Fluorionen fähige fluorhaltige Verbindung ist.

19. Anwendung nach irgendeinem der Ansprüche 1 bis 15 von mindestens einem wirkprinzip, das mindestens ein einzelliges Lebewesen, verantwortlich für sexuell übertragbare Krankheiten, hemmt oder vernichtet, und von einem Giftungs-Prinzip in Mengenverhältnissen, die dazu geeignet sind, mindestens ein mit dem besagten einzelligen Lebewesen assoziiertes Enzym zu hemmen oder zu zerstören, zur Gewinnung eines Medikaments, das zur Bekämpfung sexuell übertragbarer Krankheiten bestimmt ist.

20. Anwendung nach Anspruch 19 von Benzalkoniumchlorid und von einer fluorhaltigen Verbindung, die Fluorionen freisetzt, in geeigneten Mengenverhältnissen zur Gewinnung eines Produkts in Form eines Suppositoriums, einer Creme, einer Milch, einer Salbe, einer Pomade, eines Gels oder eines löslichen Films, das 1,20 Gew.% Benzalkoniumchlorid und 0,50 Gew.% des $F^-$-Anions enthält, oder eines Produkts in Tablettenform, das pro Tablette 25 mg Benzalkoniumchlorid und 10 mg des $F^-$-Anions enthält, oder eines Produkts in Form einer synthetischen Seife, das 2 Gew.% Benzalkoniumchlorid und 1 Gew.% des $F^-$-Anions enthält, oder eines Produkts in Form einer Lösung, das 0,50 Gew.% Benzalkoniumchlorid und 0,25 Gew.% des $F^-$-Anions enthält.

21. Anwendung nach Anspruch 19, in der ein Wirkprinzip eine zur Freisetzung von Fluorionen fähige Fluorhaltige Verbindung ist.

22. Anwendung nach irgendeinem der Ansprüche 1 bis 15 von mindestens einem Wirkprinzip, das eine antibiotische Wirkung gegenüber mindestens einem einzelligen Lebewesen besitzt, und von einem Giftungs-Prinzip in Mengenverhältnissen, die dazu geeignet sind, mindestens ein mit dem besagten einzelligen Lebewesen assoziiertes Enzym zu hemmen oder zu zerstören, zur Gewinnung eines Medikaments zur Bekämpfung derjenigen Krankheiten mittels Antibiotherapie, für die besagtes einzelliges Lebewesen verantwortlich ist.

23. Anwendung nach Anspruch 22 von Benzalkoniumchlorid oder Erythromycin-Base oder Neomycin-Base oder Bacitracin oder Oxytetracyclin-chlorhydrat oder Aureomycin-chlorhydrat oder soframycin-sulfat oder Aureomycin oder Oxytetracyclin und von einer zur Freisetzung von Fluorionen fähigen fluorhaltigen Verbindung zur Gewinnung eines Medikaments in Form einer Creme oder einer Milch oder einer Salbe oder einer Pomade, das 1,20 Gew.% Benzalkoniumchlorid und 0,50 Gew.% des $F^-$-Anions enthält, oder eines Medikaments in Form einer synthetischen Seife, das 2 Gew.% Benzalcniumchlorid und 1 Gew.% des $F^-$-Anions enthält, oder eines Medikaments in Form einer Lösung, das 0,50 Gew.% Benzalkoniumchlorid und 0,25 Gew. % des $F^-$-Anions enthält, oder eines Medikaments in Form einer Lösung oder eines Gels, das 4 Gew.% Erythromycin-Base und 0,5 Gew.% des $F^-$-Anions enthält, oder eines Medikaments in Form einer Pomade, das 0,35 Gew.% Neomycin-Base und 0,50 % dem $F^-$-Anions oder 50.000 I.E. % und 0,50 % des $F^-$-Anions oder 3 Gew. % Oxytetracyclin-chlorhydrat und 0,50 Gew.% des $F^-$-Anions oder 3 Gew.% Aureomycin-chlorhydrat und 0,50 Gew.% des $F^-$-Anions enthält, oder eines Medikaments in Cremeform, das 2,5 Gew.% Soframycin-sulfat und 0,50 Gew.% des $F^-$-Anions enthält, oder eines Medikaments in Form einer Augenpomade, das 1 Gew.% Aureomycin und 0,50 Gew.% des $F^-$-Anions oder 0,50 Gew.% Oxytetracyclin und 0,25 Gew.% des $F^-$-Anions enthält, oder eines Medikaments in Form einer Nasenlösung, das 1,25 Gew.% Soframycin-sulfat und 0,50 Gew.% des $F^-$-Anions enthält.

24. Anwendung nach Anspruch 22 von Benzalkoniumchlorid oder Erythromycin-Base oder Neomycin-Base oder Bacitracin oder Oxytetracyclin-chlorhydrat oder Aureomycin-chlorhydrat oder Soframycin-sulfat oder Aureomycin oder Oxytetracyclin und von einer zur Freisetzung von Fluorionen fähigen fluorhaltigen Verbindung zur Gewinnung eines Medikaments in Form einer Lösung oder eines Gels, das 4 Gew.% Erythromycin-Base, 0,5 Gew.% des $F^-$-Anions und 0,10 % Benzalkoniumchlorid enthält, oder eines Medikaments in Form einer Pomade, das 0,35 Gew. % Neomycin-Base, 0,50 % des $F^-$-Anions und 0,10 % Benzalkoniumchlorid oder 50.000 I.E. %, 0,50 % dem $F^-$-Anions und 0,10 % Benzalkoniumchlorid oder 3 Gew.% Oxytetracyclin-chlorhydrat, 0,50 Gew.% des $F^-$-Anions und 0,10 % Benzalkoniumchlorid oder 3 Gew.% Aureomycin-chlorhydrat, 0,50 Gew. % des $F^-$-Anions und 0,10 % Benzalkoniumchlorid enthält, oder eines Medikaments in Cremeform, das 2,5 Gew.% Soframycin-sulfat, 0,50 Gew.% des $F^-$-Anions und 0,10 % Benzalkoniumchlorid enthält, oder eines Medikaments in Form einer Augenpomade, das 1 Gew.% Aureomycin, 0,50 Gew.% des $F^-$-Anions und 0,10 % Benzalkoniumchloride oder 0,50 Gew.% Oxytetracyclin, 0,25 Gew. % des $F^-$-Anions und 0,10 % Benzalkoniumchlorid enthält, oder eines Medikaments in Form einer Nasenlösung, das 1,25 Gew.% Soframycin-sulfat, 0,50 Gew.% des $F^-$-Anions und 0,10 % Benzalkoniumchlorid enthält.

25. Anwendung nach irgendeinem der Ansprüche 1 bis 15 von mindestens einem Wirkprinzip, das mindestens einen Pilz hemmt oder vernichtet, und von einem Giftungsprinzip in Mengenverhältnissen, die dazu geeignet sind, mindestens ein mit dem besagten Filz assoziiertes Enzym zu hemmen oder zu zerstören, zur Gewinnung eines antifungiellen Produkts.

26. Anwendung von mindestens einem Wirkprinzip, das mindestens ein Pratozoon hemmt oder vernichtet, und von einem Giftungsprinzip in Mengenvernältnissen, die dazu geeignet sind, mindestens ein mit dem besagten Protozoon assoziiertes Enzym zu hemmen oder zu zerstören, zur Gewinnung eines Antiprotozoikums.

27. Anwendung von mindestens einem Wirkprinzip, das mindestens ein Bakterium hemmt oder vernichtet, und von einem GiftungsPrinzip in Mengenverhältnissen, die dazu geeignet sind, mindestens ein mit dem besagten Bakterium assoziiertes Enzym zu hemmen oder zu zerstören, zur Gewinnung eines bacteriziden Produkts oder Medikaments.

28. Anwendung nach Anspruch 27 von Benzalkoniumchlorid und von einer fluorhaltige Verbindung, die Fluorionen freisetzt, zur Gewinnung eines bakteriziden Kontaktprodukts, das 0,1 % Benzalkoniumchlorid und 0,25 % des $F^-$-Anions oder 1 % Benzalkoniumchlorid und 0,50 % des $F^-$-Anions oder 0,05 % Benzalkoniumchlorid und 0,025 % des $F^-$-Anions enthält.

29. Anwendung nach irgendeinem der Ansprüche 1 bis 15 von mindestens einem Wirkprinzip, das mindestens ein Virus oder Retrovirus hemmt oder vernichtet, und von einem Giftungsprinzip in Mengenverhältnissen, die dazu geeignet sind, mindestens ein mit dem besagten Virus oder Retrovirus assoziiertes Enzym zu hemmen oder zu zerstören, zur Gewinnung eines antiviralen Produkts oder Medikaments.